# EUROPEAN PATENT APPLICATION

(11) **EP 1 749 472 A1**
(43) Date of publication of application: **07.02.2007**
(21) Application number: 05739237.5
(22) Date of filing: 11.05.2005
(51) Int. Cl.: A61B 1/00

(54) **DEVICE FOR ACQUIRING INFORMATION IN EXAMINEE**

(30) Priority: 28.05.2004 JP 2004159823
(71) Applicant: Olympus Corporation, Tokyo 151-0072 (JP)
(72) Inventor: MORI, Takeshi, 1940201 (JP); HONDA, Takemitsu, 1910062 (JP); HOMAN, Masatoshi, 1910062 (JP)
(74) Representative: von Hellfeld, Axel
(86) International application number: PCT/JP2005/008638
(87) International publication number: WO 2005/115217

(57) **Abstract**

Image information (corresponding to 1 pixel) of 8 bits acquired by an imaging unit (23) are input into an A/D converter (24a1) provided on an AD board (23a), the input image information are digitally converted to parallel signals of 8 bits, the parallel signals are divided into every 4 bits by a bit-number-converting and outputting circuit (24a2) of an IC configuration, each parallel signals of 4 bits are output to a digital signal processor (25a1) provided on a signal processing board (25a) through four signal lines (80a) of a flexible board (80) by time division, and a predetermined signal processing is performed on the output parallel signals of 4 bits at the digital signal processor 25a1. Consequently, the number of IC pins and the number of signal lines are reduced; therefore, an capsule endoscope is miniaturized, hardening of a board is prevented, and probability of disconnection of the signal lines is reduced.

## Description

### TECHNICAL FIELD

The present invention relates to an in-vivo information acquiring apparatus such as a swallowable capsule endoscope that acquires in-vivo information while the in-vivo information acquiring apparatus is inserted into a subject.

### BACKGROUND ART

A capsule endoscope having an imaging function and a radio transmission function has entered a field of endoscopes in recent years. During an observation period from when the capsule endoscope is swallowed from a mouth of a patient, i.e., subject, for an observation (examination) until when the capsule endoscope is naturally discharged from a biological body (human body) of the patient, the capsule endoscope travels through inside organs (inside body cavity) such as a stomach and a small intestine, and sequentially images inside the organs by using the imaging function, while following peristaltic motion of the organs.

During the observation period when the capsule endoscope travels inside the organs, an image obtained inside the body by the capsule endoscope is sequentially transmitted to an external device provided outside of the subject through the radio transmission function, and stored in a memory provided in the external device. The subject can freely move during the observation period from when the capsule endoscope is swallowed until when the capsule endoscope is discharged, since the subject carries around the external device having the radio transmission function and a memory function. After the observation is finished, a diagnosis can be made by a doctor or a nurse by displaying an image, based on the image data stored in the memory of the external device, inside the body cavity on a display.

To implement the aforementioned functions, there is proposed a swallowable capsule endoscope in, for example, Patent Document 1. In the proposed capsule endoscope, an illuminator, an image sensor, a power supply unit, a transmitting unit, and the like are arranged on boards in an integrated circuit (IC) configuration, the boards are connected to others through strip boards, and these components are housed in a capsule-like airtight container.

Patent Document 1: Japanese Patent Application Laid-open No. 2001-104242

### DISCLOSURE OF INVENTION

### PROBLEM TO BE SOLVED BY THE INVENTION

Since the aforementioned capsule endoscope houses all split boards in the capsule-like container by bending the split boards, flexibility is required for the split boards. However, since the capsule endoscope transfers a plurality of digital signals indicating gradations of obtained image information through signal lines from the IC, there are problems: an increase in the number of IC pins; and in the split boards on which many signal lines are wired, an increase in a space for the components inside the capsule; loss of flexibility of the strip boards due to extended width and increased layers, and an increase in probability of disconnection of signal lines.

The present invention is provided in view of the foregoing, and an object of the present invention is to provide an in-vivo information acquiring apparatus in which the number of IC pins and the number of signal lines are reduced. Consequently, a capsule endoscope can be miniaturized, hardening of a board can be prevented, and probability of disconnection of the signal lines can be reduced.

### MEANS FOR SOLVING PROBLEM

In order to solve the aforementioned problems and to achieve the object, an in-vivo information acquiring apparatus according to the present invention is inserted into a subject to acquire information inside the subject, and includes a function executing unit that performs a predetermined function for acquiring the information inside the subject; a signal output unit that divides and outputs the information acquired by the function executing unit; and a signal processor that performs a signal processing on the information output by the signal output unit.

The in-vivo information acquiring apparatus according to the invention as set forth in claim 2 includes a plurality of rigid boards in which each of the function executing unit, the signal output unit, and the signal processor is arranged on each different one of the rigid boards, respectively; a flexible board that is arranged between the rigid boards and transfers the information; and an exterior case that houses the rigid boards and the flexible board and is liquidtight inside.

In the in-vivo information acquiring apparatus according to the invention as set forth in claim 3, the signal output unit includes a signal converter that converts the information acquired by the function executing unit to a digital signal, and a dividing unit that divides and outputs the digital signal converted by the signal converter.

In the in-vivo information acquiring unit according to the invention as set forth in claim 4, the dividing unit divides the digital signal converted by the signal converter by a predetermined parallel bit number, and outputs each divided digital signal by time division.

In the in-vivo information acquiring apparatus according to the invention as set forth in claim 5, the function executing unit includes at least an illuminating unit that outputs illuminating light illuminating an inside of the subject, an imaging unit that acquires image information inside the subject illuminated by the illuminating light emitted from the illuminating unit, and a radio transmitting unit that radio-transmits the image information acquired by the imaging unit to outside.

### EFFECT OF THE INVENTION

The in-vivo information acquiring apparatus according to the present invention divides, for example, parallel bit information acquired by the function executing unit, and outputs by time division each divided parallel bit information to the rigid board on which a signal processor is arranged, through the flexible board. Consequently, the number of signal lines can be reduced; therefore, hardening of the board can be prevented, and probability of disconnection of the signal lines can be reduced.

### BRIEF DESCRIPTION OF DRAWINGS

FIG. 1 is a schematic diagram of an overall configuration of a wireless in-vivo information acquiring system including an in-vivo information acquiring apparatus according to the present invention;
FIG. 2 is a sectional side view of a configuration of a first embodiment of the in-vivo information acquiring apparatus according to the present invention;
FIG. 3 is a top view in which a rigid/flexible wiring board shown in FIG. 2 is developed;
FIG. 4 is a bottom view in which the rigid/flexible wiring board shown in FIG. 2 is developed;
FIG. 5 is a block diagram of a relevant configuration, according to the present invention, of a driving IC and an image processing IC shown in FIG. 2; and
FIG. 6 shows output timing of digital data at a bit-number-converting and outputting unit shown in FIG. 5.

### EXPLANATIONS OF LETTERS OR NUMERALS

- 1: Subject
- 2: Capsule endoscope
- 3: Receiving device
- 4: Display device
- 5: Portable recording medium
- 6: Airtight container
- 8: Rigid/flexible wiring board
- 20: Illuminating unit
- 20a: Illuminating board
- 20b: Through hole
- 20c: Light emitters
- 20d: Chip pieces
- 23: Imaging unit
- 23a: AD board
- 23b: Solid-state image sensor
- 23c: Focusing lens
- 23d: Focus adjusting mechanism
- 24: Driving and signal output unit
- 24a: Driving IC
- 24a1: A/D converter
- 24a2: Bit-number-converting and outputting unit
- 24b: Chip pieces
- 25: Signal processor
- 25a: Signal processing board
- 25b: Image processing IC
- 25b1: Digital signal processor
- 25c: Contact point
- 27: Radio transmitting unit
- 27a: Transmitting board
- 27b: Oscillating circuit
- 27c: Antenna board
- 27d: Antenna
- 30: Accumulating unit
- 30a: Power supply board
- 30b: Button type battery
- 30c: Power supply unit
- 30c1: Converter
- 31: Receiving jacket
- 32: External device
- 41: Solid-state image sensor
- 61: Front cover
- 62: Body portion cover
- 63: Body portion
- 64: Rear end portion
- 80: Flexible board
- 80a: Signal lines

### BEST MODE(S) FOR CARRYING OUT THE INVENTION

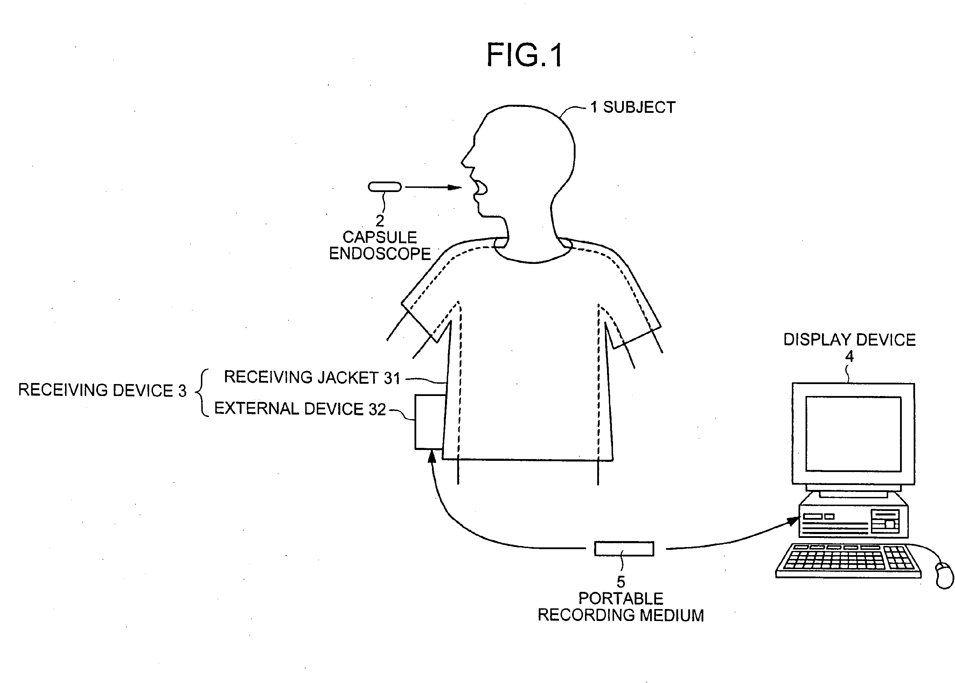

Embodiments of an in-vivo information acquiring apparatus according to the present invention will be described in detail below with reference to FIGS. 1 to 6. The present invention is not limited to the embodiments, and various modifications may be made without departing from the spirit or scope of the general inventive concept as defined by the appended claims and their equivalents.

### FIRST EMBODIMENT

FIG. 1 is a schematic diagram of an overall configuration of a wireless in-vivo information acquiring system including an in-vivo information acquiring apparatus according to the present invention. In the wireless in-vivo information acquiring system, an capsule endoscope, which is inserted into a body cavity from a mouth of a human, i.e., subject, and images an examined region inside the body cavity, is explained as an example of the in-vivo information acquiring apparatus. In FIG. 1, the wireless in-vivo information acquiring system includes a receiving device 3 that has a radio receiving function, and an capsule endoscope (in-vivo information acquiring apparatus) 2 that is inserted into a subject 1, obtains an image inside the body cavity, and transmits data such as image signals to the receiving device 3. Further, the wireless in-vivo information acquiring system includes a display device 4 that displays the image inside the body cavity based on the image signals received by the receiving device 3, and a portable recording medium 5 that transfers the data between the receiving device 3 and the display device 4. The receiving device 3 includes a receiving jacket 31 that is worn by the subject 1, and an external device 32 that performs, for example, a processing on the received radio signals.

The display device 4 serves to display the image, which is obtained by the capsule endoscope 2, inside the body cavity, and has a configuration such as a work station that displays the image based on the data acquired from the portable recording medium 5. Specifically, the display device 4 may directly display the image through a cathode ray tube (CRT) display, a liquid crystal display, and the like, or may output the image to other medium as in a printer.

The portable recording medium 5 is detachable with respect to the external device 32 and the display device 4, and can record or output information when the portable recording medium 5 is attached to one of the external device 32 and the display device 4. In the embodiment, the portable recording medium 5 is attached to the external device 32 and records the data transmitted from the capsule endoscope 2, while the capsule endoscope 2 travels inside the body cavity of the subject 1. After the capsule endoscope 2 is discharged from the subject 1, which is after the imaging inside the subject 1 is finished, the portable recording medium 5 is removed from the external device 32, and attached to the display device 4. Then, the display device 4 reads the data recorded on the portable recording medium 5. Unlike when the external device 32 and the display device 4 are directly connected to each other through a cable, the subject 1 can freely move while the capsule endoscope 2 images inside the body cavity, since the data are transferred between the external device 32 and the display device 4 through a portable recording medium 5 consisting of a CompactFlash® memory and the like. The manner of data transfer between the external device 32 and the display device 4 in the present invention is not limited to the use of the portable recording medium 5. For example, other recording device such as a hard disc may be installed in the external device 32, and the internal recording device and the display device 4 may be connected through a wireless connection or through a cable to transfer the data.

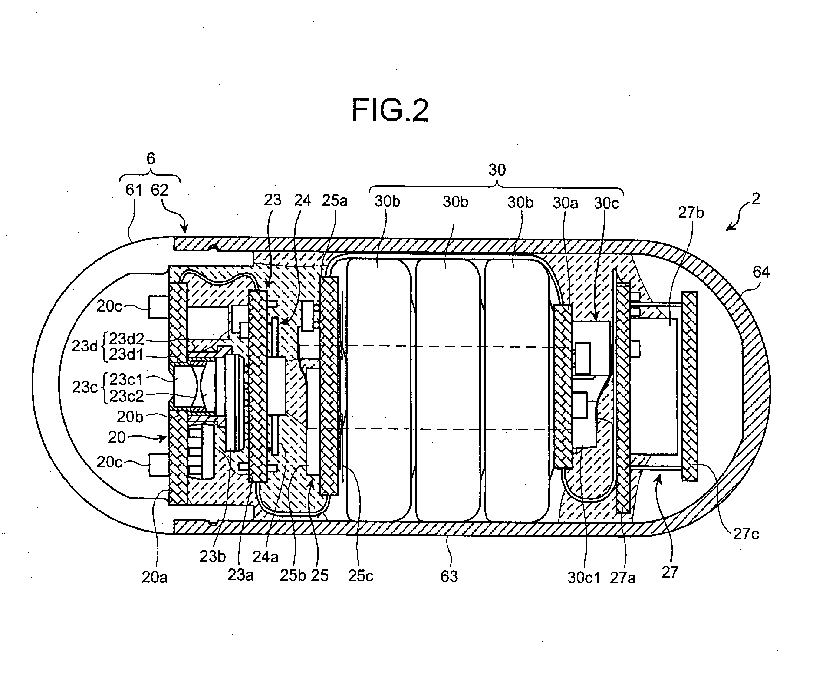

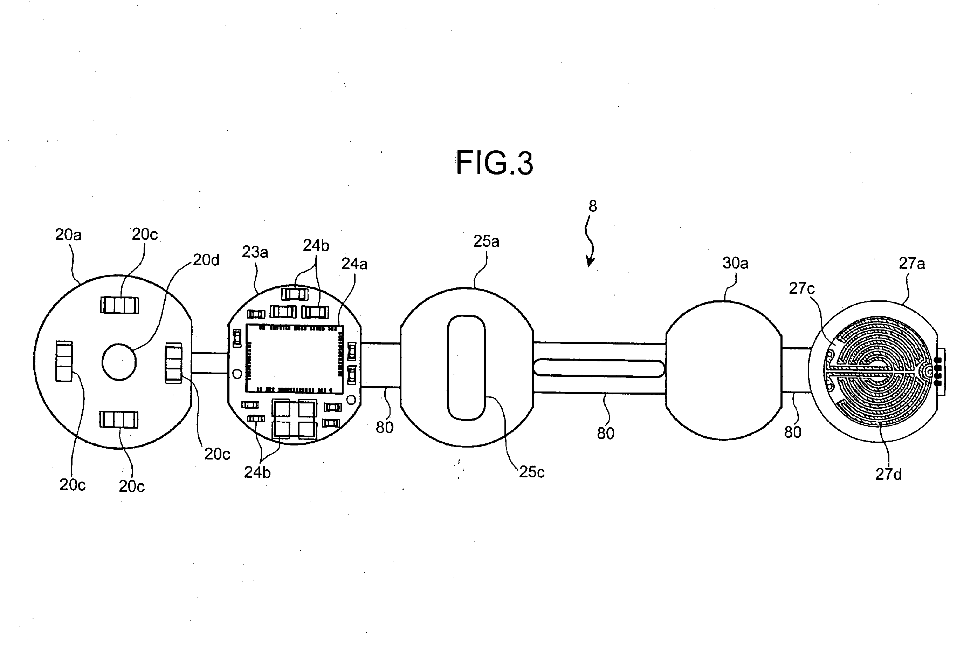

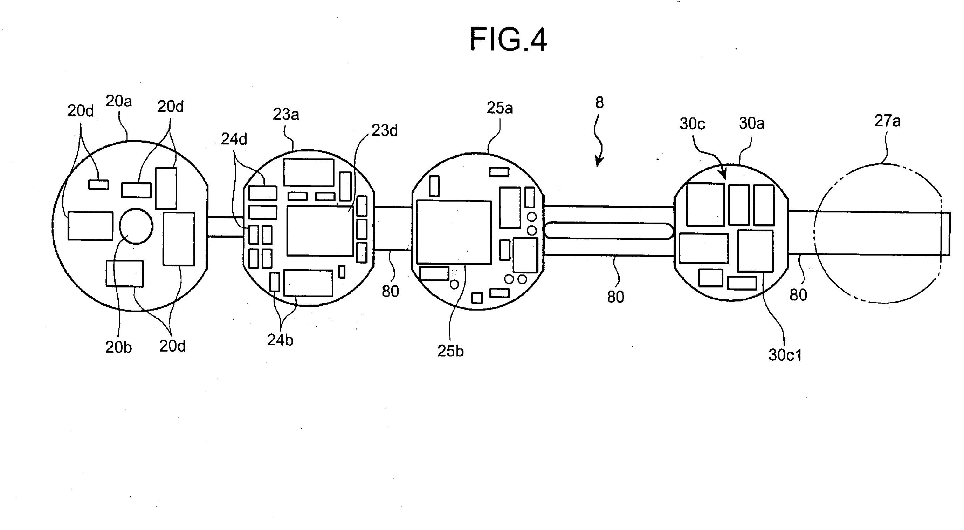

FIG. 2 is a sectional side view of a configuration of a first embodiment of the in-vivo information acquiring apparatus according to the present invention. FIG. 3 is a top view in which a rigid/flexible wiring board shown in FIG. 2 is developed. FIG. 4 is a bottom view in which the rigid/flexible wiring board is developed. In the embodiment, an capsule endoscope, which is inserted into a body cavity from a mouth of a human, i.e., subject, and images an examined region inside the body cavity, is explained as an example of the in-vivo information acquiring apparatus.

As shown in FIG. 2, the capsule endoscope 2 has an airtight container 6, an illuminating unit 20, an imaging unit 23, a driving-and-signal output unit 24, a signal processor 25, a accumulating unit 30, and a radio transmitting unit 27. The airtight container 6 is an exterior case having a capsule shape. The illuminating unit 20 is a function executing unit for implementing a predetermined function, and illuminates the examined region inside the body cavity with illuminating light. The imaging unit 23 is a function executing unit, and receives reflected light of the illuminating light to image the examined region. The driving-and-signal output unit 24 performs driving controlling of the illuminating unit 20 and the imaging unit 23, and performs signal conversion. The signal processor 25 performs a signal processing. The accumulating unit 30 accumulates driving power for driving the function executing units. The radio transmitting unit 27 is a function executing unit, and radio-transmits the image data acquired by the imaging unit 23 to outside of the subject.

The airtight container 6 has a human swallowable size, and consists of a substantially semispherical front cover 61 and a cylindrical body portion cover 62 elastically fit with each other. An illuminating board 20a, an analog-to-digital (AD) board 23a, a signal processing board 25a, a power supply board 30a, and a transmitting board 27a are inserted into the cylindrical body portion cover 62 in which a rear end portion thereof has a substantially semispherical base portion and a distal end portion thereof has a circular opening. The front cover 61 has a substantially semispherical dorm shape, and a rear side of the dorm is opened circularly. The front cover 61 is formed by a transparent material, which is preferred to be used to obtain optical property and strength, such as the cycloolefin polymer or the polymer carbon having transparency or translucency. Hence, the front cover 61 allows the illuminating light emitted from the illuminating unit 20 to transmit to outside of the airtight container 6, and allows the reflective light emitted from the subject due to the illuminating light to transmit to the interior of the airtight container 6.

The body portion cover 62 is arranged at the rear side of the front cover 61, and covers the aforementioned function executing units. A cylindrical body portion 63 and a substantially spherical dorm-like rear end portion 64 are integrated with each other to form the body portion cover 62, and a front side of the body portion 63 is opened circularly. The body portion cover 62 is formed by the polysulfone and the like, which is preferred to be used to obtain strength. In the body portion cover 62, the body portion 63 houses the illuminating unit 20, the imaging unit 23, the driving-and-signal output unit 24, the signal processor 25, and the accumulating unit 30, and the rear end portion 64 houses the radio transmitting unit 27.

As shown in FIGS. 2 and 3, the illuminating unit 20 has a discoidal illuminating board 20a and four light emitters 20c, and as shown in FIG. 4, the illuminating unit 20 has chip pieces 20d. The illuminating board 20a has a through hole 20b at a central region thereof. The light emitters 20c are light-emitting diodes (LED) such as white LEDs provided on a front face (in FIG. 2, front cover 61 side) of the illuminating board 20a. The chip pieces 20d are arranged on a back face (in FIG. 2, AD board side), and configures a circuit for driving the light emitters 20c. The light emitters 20c illuminate outside with the illuminating light emitted through the front cover 61.

As shown in FIGS. 2 and 4, the imaging unit 23 has a discoidal AD board 23a, a solid-state image sensor 23b, and a focusing lens 23c. The solid-state image sensor 23b is a charge-coupled device (CCD), a complementary metal oxide semiconductor (CMOS), or the like provided on a front face (in FIG. 2, illuminating board 20a side) of the AD board 23a. The focusing lens 23c focuses the image of the subject on the solid-state image sensor 23b. The focusing lens 23c is provided on a front face (in FIG. 2, illuminating board 20a side) of the solid-state image sensor 23b, and has a first lens 23c1 and a second lens 23c2. The first lens 23c1 is arranged at an object side, and provided in a securing frame 23d1. The second lens 23c2 is arranged at a solid-state image sensor 23b side, and provided in a moving frame 23d2. The securing frame 23d1 and the moving frame 23d2 configure a focus adjusting mechanism 23d that shifts the second lens 23c2 along an optical axis. The securing frame 23d1 penetrates through the through hole 20b of the illuminating board 20a, and maintains the optical axis of the focusing lens 23c to point towards the front face of the illuminating board 20a. Consequently, the imaging unit 23 can image a region illuminated by the illuminating light emitted from the illuminating unit 20.

As shown in FIGS. 2 and 3, the driving-and-signal output unit 24 has a driving IC 24a and chip pieces 24b. The driving IC 24a is provided on a back face (in FIG. 2, signal processing board 25a side) of the AD board 23a, and configures a circuit for driving the solid-state image sensor 23b. The chip pieces 24b are arranged so as to enclose the driving IC 24a and the solid-state image sensor. 23b. The driving IC 24a has a central role in a driving controlling of the capsule endoscope 2, and has a driving controlling circuit, not shown, performing a driving controlling of the solid-state image sensor 41, an output signal processing, and a driving controlling of the illuminating unit 20. The chip pieces 24b provided at the back face of the AD board 23a are semiconductor materials having a mixing function. The mixing function mixes two signals of clock signals and image signals (image information) output from the driving IC 24a to produce one signal, to transmit the clock signals and the image signals from the radio transmitting unit 27. The driving IC 24a has an analog/digital (A/D) converter 24a1 and a bit-number-converting and outputting unit 24a2, which are signal output units described later.

As shown in FIGS. 2 and 4, the signal processor 25 has a discoidal signal processing board 25a and an image processing IC 25b. The image processing IC 25b is provided on a front face (in FIG. 2, AD board 23a side) of the signal processing board 25a, and performs an information processing on the image information. The image processing IC 25b has a digital signal processor 25b1 described later. The signal processor 25 has a contact point 25c formed by a plate spring provided on a back face (in FIG. 2, power supply board 30a side) of the image processing board 25a. The contact point 25c contacts an anode of the button type battery 30b described later, so that the button type battery 30b is pressed towards the back side (in FIG. 2, power supply board 30a side) due to force of the plate spring.

As shown in FIG. 2, the accumulating unit 30 has a discoidal power supply board 30a, the button type batteries 30b such as silver oxide batteries arranged between the signal processing board 25a and the power supply board 30a, and a power supply unit 30c. A plurality of the button type batteries 30b, i.e., three button type batteries 30b in the present embodiment are arranged in series, and a cathode cap side thereof is arranged so as to face towards the back side. The battery 30b is not limited to the silver oxide battery; therefore, for example, a rechargeable battery, a power generating battery, and the like may be utilized. Further, the number of batteries 30b is not limited to three.

The power supply unit 30c has a DC/DC (direct current/direct current) converter 30c1 provided on a back face (in FIG. 2, rear end portion 64 side) of the power supply board 30a. The DC/DC converter 30c1 controls, for example, a step up of a voltage acquired from the button type batteries 30b to constantly acquire a steady voltage necessary for the system. A contact point that is in contact with the cathode cap of the button type battery 30b, not shown, is provided on a front side (in FIG. 2, signal processing board 25a side) of the power supply board 30a. In the embodiment, the plurality of the button type batteries 30b are connected in series between the signal processing board 25a and the power supply board 30a. Consequently, the accumulating unit 30 can supply power to each function executing unit.

As shown in FIGS. 2 and 3, the radio transmitting unit 27 has a discoidal transmitting board 27a, an oscillating circuit 27b provided on a back face (in FIG. 2, rear end portion 64 side) of the transmitting board 27a, an antenna board 27c, and an antenna 27d provided on a back face (in FIG. 2, rear end portion 64 side) of the antenna board 27c. As shown in FIG. 3, the antenna 27d is arranged on the back side of the antenna board 27c in a substantially spiral shape. In the radio transmitting unit 27, the oscillating circuit 27b extracts signals having particular frequency, amplitude, and waveform from the signals mixed at the aforementioned chip pieces 24b (semiconductor material), and the extracted signals are transmitted to outside of the capsule endoscope 2 from the antenna 27d. The transmitting board 27a and the antenna board 27c are electrically connected to each other by soldering, and configure one transmitting unit.

The aforementioned illuminating board 20a, the AD board 23a, the signal processing board 25a, the power supply board 30a, and the transmitting board 27a consist of rigid boards. As shown in FIGS. 3 and 4, the rigid boards are provided so as to sandwich lines of flexible boards 80 thereamong, and the rigid boards and the flexible boards 80 configures a rigid/flexible wiring board 8. The illuminating board 20a, the AD board 23a, the signal processing board 25a, the power supply board 30a, and the transmitting board 27a are arranged in this order with a predetermined spacing while having the flexible boards 80 thereamong, and each rigid board is electrically connected to each other. The flexible boards 80 of the rigid/flexible wiring board are bent so as to layer the illuminating board 20a, the AD board 23a, the signal processing board 25a, the power supply board 30a, and the transmitting board 27a in a backward and forward directions towards the front cover 61 side and the rear end portion 64 side, as shown in FIG. 1.

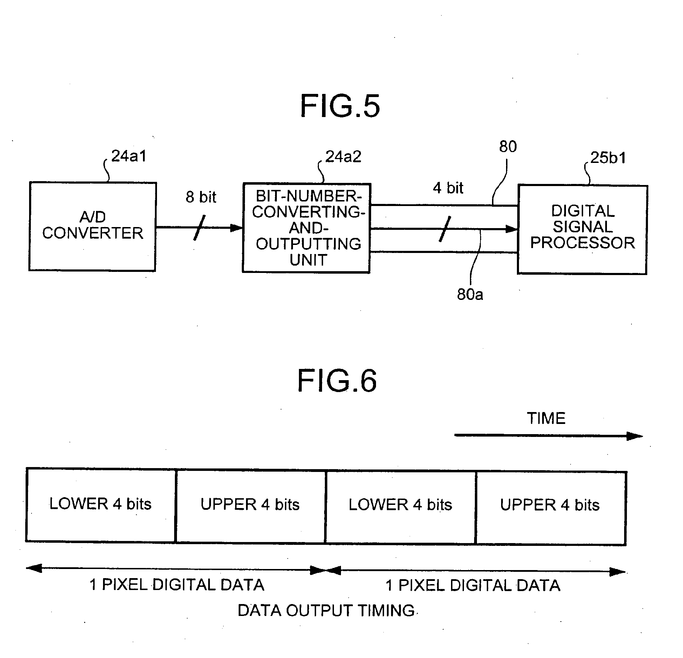

FIG. 5 is a block diagram of a relevant configuration, according to the present invention, of the driving IC and the image processing IC shown in FIG. 2. In FIG. 5, the driving IC 24a includes the A/D converter 24a1 being a signal converter, and the bit-number-converting and outputting unit 24a2 being a dividing unit into which the image information such as parallel signals of 8 bit transferred from the A/D converter 24a1 are input. The image processing IC 25b includes a digital signal processor 25b1, and the bit-number-converting and outputting unit 24a2 and the digital signal processor 25b1 are connected to each other through the flexible board 80 on which four signal lines 80a are wired.

In accordance with the aforementioned configurations, the image information (digital data) corresponding to 1 pixel acquired at the imaging unit 23 is input into the A/D converter 24a1 provided on the AD board 23a. The A/D converter 24a1 performs a digital conversion on the input image information, and the input image information becomes the parallel signals of 8 bits. Then, the parallel signals of 8 bit are divided into parallel signals of upper 4 bits and lower 4 bits at the bit-number-converting and outputting unit 24a2. The bit-number-converting and outputting unit 24a2 outputs each aforementioned divided parallel signals by time division to the digital signal processor 25b1 provided on the signal processing board 25a through the four signal lines 80a provided on the flexible board 80. As shown in FIG. 6 for example, in the bit-number-converting and outputting unit 24a2, the image information corresponding to 1 pixel are output as digital data of upper 4 bits and digital data of lower 4 bits, sequentially by time division, to the four signal lines 80a. The digital signal processor 25b1 performs a predetermined signal processing on the input digital data, and can output the digital data, on which the predetermined digital processing is performed, to the transmitting board 27a through the flexible boards 80 and the power supply board 30a.

As described hereinbefore, in the present embodiment, the image information of 8 bits acquired at the imaging unit are divided into parallel signals of every 4 bits at the bit-number-converting and outputting circuit of the IC configuration provided on the AD board, and output to the digital signal processor provided on the signal processing board through the four signal lines arranged on the flexible board. Consequently, the number of IC pins arranged on the rigid boards and the number of the signal lines arranged on the flexible boards can be reduced. Hence, the capsule endoscope can be miniaturized, and further, decrease in the widths and layers of the boards prevents hardening thereof, so that bending of the boards can easily be performed, and probability of disconnection of the signal lines can be reduced.

In the present embodiment, the image information are divided for every 4 bits. However, the present invention is not limited thereto, and the image information can be divided into, for example, parallel signals of every 2 bits. Hence, only two signal lines are required to be wired on the flexible boards, so that the hardening of the boards can further be prevented, and the probability of the disconnection of the signal lines can be reduced. The image information can be divided for every 1 bit. When the image information is divided by a serial signal of every 1 bit, the number of the signal lines wired on the flexible boards becomes one. However, for example, when a frame rate of outputting two frames in 1 second is maintained, a driving frequency thereof becomes 8 times larger than a frequency of a reference clock. Consequently, current consumption is largely increased, and battery duration is largely shortened. Hence, the present invention is explained in view of the aforementioned embodiment, so that the current frame rate is maintained, the driving frequency is not largely increased, and the number of signal lines can be reduced.

The present invention is not limited to outputting the image information, and the present invention can be applied to when other digital data are output, i.e., to when parameter information such as white data are output. Further, in the present invention, the parameter information and the image information can be output by time division.

### INDUSTRIAL APPLICABILITY

As described hereinbefore, a body insertable apparatus according to the present invention is useful for a medical observation apparatus, such as an capsule endoscope that is inserted into a human body and observes an examined region. Particularly, the body insertable apparatus is suitable to reduce the number of IC pins and the number of signal lines, so that the capsule endoscope is miniaturized, hardening of a board is prevented, and probability of disconnection of signal line is reduced.

## Claims

1. An in-vivo information acquiring apparatus that is inserted into a subject and acquires information inside the subject, the in-vivo information acquiring apparatus comprising:
a function executing unit that executes a predetermined function for acquiring the information inside the subject;
a signal output unit that divides and outputs the information acquired by the function executing unit; and
a signal processor that performs a signal processing on the information output by the signal output unit.

2. The in-vivo information acquiring apparatus according to claim 1, further comprising:
a plurality of rigid boards in which each of the function executing unit, the signal output unit, and the signal processor is arranged on each different one of the rigid boards;
a flexible board that is arranged between the rigid boards and transfers the information; and
an exterior case that houses the rigid boards and the flexible board and is liquidtight inside.

3. The in-vivo information acquiring apparatus according to claim 1 or 2, wherein
the signal output unit includes
a signal converter that converts the information acquired by the function executing unit to a digital signal, and
a dividing unit that divides and outputs the digital signal converted by the signal converter.

4. The in-vivo information acquiring unit according to claim 3, wherein
the dividing unit divides the digital signal converted by the signal converter, by a predetermined parallel bit number, and outputs each divided digital signal by time division.

5. The in-vivo information acquiring apparatus according to claim 1 or 2, wherein
the function executing unit includes at least
an illuminating unit that outputs illuminating light illuminating an inside of the subject,
an imaging unit that acquires image information inside the subject illuminated by the illuminating light emitted from the illuminating unit, and
a radio transmitting unit that radio-transmits the image information acquired by the imaging unit to outside.
